(19) Europäisches Patentamt
European Patent Office
Office européen des brevets

(11) **EP 4 682 569 A1**

(12) **EUROPEAN PATENT APPLICATION**

(43) Date of publication:
**21.01.2026 Bulletin 2026/04**

(21) Application number: **24382785.4**

(22) Date of filing: **18.07.2024**

(51) International Patent Classification (IPC):
**G01R 33/26** (2006.01)    **G01N 24/00** (2006.01)

(52) Cooperative Patent Classification (CPC):
**G01R 33/26; G01N 24/006; A61B 5/245**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(71) Applicants:
• **Fundació Institut de Ciències Fotòniques**
  **08860 Castelldefels (Barcelona) (ES)**
• **Institució Catalana de Recerca i Estudis Avançats**
  **08010 Barcelona (ES)**

(72) Inventors:
• **Raghavan, Harini**
  **08860 CASTELLDEFELS (ES)**
• **Mouloudakis, Kostas**
  **08860 CASTELLDEFELS (ES)**
• **Mitchell, Morgan**
  **08860 CASTELLDEFELS (ES)**
• **Tayler, Michael**
  **08860 CASTELLDEFELS (ES)**

(74) Representative: **Grünecker Patent- und Rechtsanwälte**
**PartG mbB**
**Leopoldstraße 4**
**80802 München (DE)**

(54) **DETECTION OF ZERO-FIELD RESONANCE**

(57) The present invention relates to a method of detecting a zero-field resonance (ZFR), comprising the steps of i) directing a pump light beam in a first direction x through a vapor cell comprising gaseous atoms, ii) applying a magnetic field component to the vapor cell parallel or antiparallel to the first direction x with different magnitudes $B_{x,i}^{app}$, iii) detecting light of the pump light beam directed through the vapor cell for the different magnitudes $B_{x,i}^{app}$ of the magnetic field component applied parallel or antiparallel to the first direction x, iv) determining the maximum optical transmission $T_{max}$ and the minimum optical transmission $T_{min}$ based on the detected light, v) determining the difference between the maximum optical transmission and the minimum optical transmission $D = T_{max} - T_{min}$, vi) determining the magnitude of the x-component of an ambient magnetic field $B_x^{ext}$ by determining the particular magnitude $B_x^{app}$ of the x-component of the applied magnetic field for which the minimum optical transmission $T_{min}$ is determined, vii) repeating at least steps i) to v) while applying magnetic field components to the vapor cell in second and third directions y and z perpendicular to the first direction x with different magnitudes $B_{y,i}^{app}$ and $B_{z,i}^{app}$, and viii) determining the magnitudes $B_y^{ext}$ and $B_z^{ext}$ of the ambient magnetic field components in the second and third directions y and z by determining the particular magnitudes of the y- and z-components of the applied magnetic field $B_y^{app}$ and $B_z^{app}$ for which the difference between the maximum optical transmission and the minimum optical transmission D is minimized.

Fig. 1

## Description

### Field of Invention

The present invention relates to a method and an apparatus for detecting zero-field resonance and, in particular, magnetometers employing the zero-field resonance effect.

### Background of the invention

Zero-field resonance (ZFR) is a phenomenon in which an atomic medium is largely transparent to laser light at zero or near-zero magnetic field and is largely opaque to laser light at other values of the magnetic field. The transparency range might be, for example, -100 nT to +100 nT in any of the three vector components of the magnetic field. For comparison, the field of the earth is about 50 $\mu$T or 50,000 times larger than the transparency range.

Employing the zero-field resonance effect has a wide and growing range of applications including the detection of biomagnetic signals, neuroscience tools, geophysical exploration and prospecting, navigation and space applications as well as microwave communication, in general.

Particularly, the zero-field resonance effect can be employed by highly sensitive magnetometers that measure the direction and magnitude of an external magnetic field through the induced changes in the atomic spin polarization of an ensemble of atoms. In particular, optically pumped magnetometers (OPMs) operate based on the zero-field resonance effect. An optically pumped magnetometer is a device that uses lasers and an atomic medium to obtain an optical signal that indicates the magnetic field of interest. Typically, an OPM operating based on ZFR incorporates magnetic field coils arranged around the atomic medium and uses active feedback (biasing by means of the magnetic coils) to maintain the magnetic field at the sensed location within the transparency range. Thus, in the active feedback application the OPM must generate a field that cancels the ambient field or a residual ambient magnetic field still present after some magnetic field shielding which is not known beforehand but should be determined by the OPM. Therefore, the magnetic field applied by the magnetic coils is to be scanned over a range of values in the attempt of finding the correct strengths of the individual magnetic field components to compensate for the ambient field (thus "finding the zero" of the field). This search is complicated, particularly, since it has to be performed in all three spatial dimensions.

US 2016/0223627 A1 proposes a solution for the search problem based on the application of a bias magnetic field in the direction of the laser pump beam to simultaneously increase both the height and width of the ZFR. Subsequently, the ZFR is detected and a magnetic field is scanned/swept in directions perpendicular to that of the pump light beam (transversal directions of the magnetic field) and the transversal magnetic field components generated by one or more magnetic coils are adjusted to minimize the width and maximize the height of the zero-field resonance (transmission). However, this method requires precise adjustment of the applied magnetic field components relative to the ambient magnetic field and the ZFR width. It also involves detecting small changes in relatively large measurement quantities. The procedure, therefore, is rather time-consuming.

It is, therefore, an object of the present invention to provide a technique for employing the zero-field resonance effect with high accuracy and at high operation speed, particularly, in the context of magnetometer applications.

### Description of the Invention

The present invention addresses the above-mentioned object by providing a method of detecting a zero-field resonance (ZFR), comprising the step of i) directing a pump light beam in a first direction x through a vapor cell comprising gaseous atoms. The gaseous atoms are or comprise one of alkali atoms, rubidium atoms, cesium atoms, potassium atoms, sodium atoms and helium atoms. The vapor cell may, additionally, contain a buffer gas provided to slow down the rate at which the atoms collide with the inner walls of the vapor cell such that randomization of the spins of the atoms caused by such collisions can be suppressed.

The method further comprises the steps of ii) applying (by means of a magnetic field coil) a (longitudinal) magnetic field component to the vapor cell parallel or antiparallel to the first direction x with different magnitudes $B_{x,i}^{\mathrm{app}}$ (i = 1, .., N, N being an integer indicating the number of different magnitudes), iii) detecting light of the pump light beam directed through the vapor cell for the different magnitudes $B_{x,i}^{\mathrm{app}}$ of the magnetic field component applied parallel or antiparallel to the first direction x (i.e., sweeping/scanning the applied longitudinal magnetic field component) and iv) determining (for the different N magnitudes of the applied longitudinal magnetic field) the maximum optical transmission $T_{max}$ and the minimum optical transmission $T_{min}$ based on the detected light. Then, in step v) the difference between the maximum optical transmission and the minimum optical transmission $D = T_{max} - T_{min}$ (i.e., the depth of the optical transmission dip) is determined.

The method further comprises vi) determining the magnitude of the x-component of an ambient magnetic field $B_x^{\mathrm{ext}}$ by determining the particular magnitude $B_x^{\mathrm{appl}}$ of the x-component of the applied magnetic field for which the minimum optical transmission $T_{min}$ is determined. Here and in the following the ambient magnetic field is an external naturally occurring magnetic field or a residual external naturally occurring magnetic field that remains after shielding the vapor cell by some magnetic shielding. This magnitude of the x-component of an ambient magnetic field $B_x^{ext}$ may be considered to be equal to the particular one of the magnitudes of the applied longitudinal magnetic field component for which the minimum transmission $T_{min}$ is determined.

$T_{max}$, $T_{min}$ and, therefore, D depend on (transversal) magnetic field components applied to the vapor cell in second and third directions y and z perpendicular to the first direction x. Therefore, the method, furthermore, comprises vii) repeating at least steps i) to v) (with possibly varying N) while applying (transversal) magnetic field components to the vapor cell in second and third directions y and z perpendicular to the first direction x with different magnitudes $B_{y,i}^{app}$ and $B_{z,i}^{app}$, and viii) determining the magnitudes $B_y^{ext}$ and $B_z^{ext}$ of the ambient magnetic field components in the second and third directions y and z by determining the particular magnitudes of the y- and z-components of the applied magnetic field $B_y^{app}$ and $B_z^{app}$ for which the difference between the maximum optical transmission and the minimum optical transmission D is minimized. The magnitudes $B_y^{ext}$ and $B_z^{ext}$ of the ambient magnetic field components are considered to be equal to or are determined based on the particular magnitudes $B_y^{app}$ and $B_z^{app}$ of the applied transversal magnetic field components.

It is noted that when carrying out step ii) the first time before the repetition cycle vii) the y- and z-components of the applied magnetic field may be chosen to be zero, for example. Further, when repeating step ii) during the repetition cycle vii) the number of different magnitudes N of the applied longitudinal magnetic field component may be varied with respect to step ii) carried out for the first time before the repetition cycle vii). According to a particular example, N=1 during the repetition cycle vii) and the only applied longitudinal magnetic field component has the particular magnitude $B_x^{app}$ determined in step vi).

It is noted that step vi) of determining the magnitude of the x-component of an ambient magnetic field $B_x^{ext}$ by determining the particular magnitude $B_x^{app}$ of the x-component of the applied magnetic field for which the minimum optical transmission $T_{min}$ is determined may also be repeated through the cycle of step vii) and, for example, the x-component of an ambient magnetic field $B_x^{ext}$ may be determined as an average of the particular magnitudes $B_x^{app}$ of the x-component of the applied magnetic field for which the minimum optical transmission $T_{min}$ is determined for different magnitudes of the y- and z-components of the applied magnetic field $B_y^{ext}$ and $B_z^{ext}$.

This method allows for a fast and reliable determination of the ambient magnetic field and, thus, the ZFR. The sweeping/scanning of the applied longitudinal magnetic field component while observing the transmission allowing for determining optical transmission dips with depths D is essential for the present invention. The inventive method is based on a minimization of D such that all nonzero values of D are significant and, consequently, optimization can be performed speedily without high precision measurements that rely on small differences between relatively large finite values rather than differences with respect to zero (see also detailed description below) as needed in the prior art. Scan and overall processing speed can be increased as compared to the art. Particularly, according to the inventive method there is no need for a computationally expensive fitting of the width of the ZFR or any time expensive modulation/demodulation processing. Further, accuracy of the determined ambient magnetic field is increased by the inventive method as compared to the art. According to an implementation, the magnitudes $B_{y,i}^{app}$ and $B_{z,i}^{app}$ of the applied transversal magnetic field components in the second and third directions y and z are varied in step vii) based on at least one of a raster scan, spiral search starting from a starting magnitude and moving outward in a spiral pattern, Lissajous search with magnitudes oscillating sinusoidally at different frequencies, a Rose curve search by oscillating the transversal applied magnetic field magnitudes $\sqrt{\left(B_y^{app}\right)^2 + \left(B_z^{app}\right)^2}$ sinusoidally while linearly scanning the angle $\arctan\left(B_z^{app}/B_y^{app}\right)$ and a sample of representative points. These kinds of searches allow for a speedy and reliable adjustment of the applied transversal magnetic field components to the actual transversal magnetic field components of the ambient magnetic field in order to determine the ZFR.

According to an implementation, in step viii) the magnitudes $B_y^{ext}$ and $B_z^{ext}$ of the transversal ambient magnetic field components are determined by weighted averages of the magnitudes $B_{y,i}^{app}$ and $B_{z,i}^{app}$ of the applied transversal magnetic field components wherein the weights are functions of the determined optical transmissions. This weighting procedure allows for a fast and reliable determination of the magnitudes $B_y^{ext}$ and $B_z^{ext}$ of the transversal ambient magnetic field components. For example, the magnitudes $B_y^{ext}$ and $B_z^{ext}$ are determined by

$$B_y^{ext} = \sum_{i=1}^{n} B_{y,i}^{app} \; w_i,$$

$$B_z^{ext} = \sum_{i=1}^{n} B_{z,i}^{app} \; w_i,$$

with

$$w_i = \begin{cases} \left(\dfrac{T_i}{T_{max}}\right)^x & T_i > v \; T_{max} \\ \quad 0 & \text{otherwise} \end{cases}$$

wherein $T_{max}$ is the maximum value of the determined transmission $T_i$, $x$ is a power-law exponent, $v$ is the threshold fraction and $i = 1, .., n$, $n$ being an integer indicating the number of different magnitudes.

According to an implementation, the method further comprises generating the pump light beam by a laser device. Alternatively, the pump light beam may be generated by a suitable vapor lamp.

The above-mentioned object is also addressed by providing an apparatus for detecting a zero-field resonance, comprising a vapor cell comprising gaseous atoms (for example, being or comprising one of alkali atoms, rubidium atoms, cesium atoms, potassium atoms, sodium atoms and helium atoms), a light source configured to direct a pump light beam in a first direction x through the vapor cell, magnetic coils arranged and configured to apply a magnetic field to the (interior of the) vapor cell, a detector device configured to detect light of the pump light beam directed through the vapor cell, and a processing and controlling means. The processing and controlling means is configured to perform

    I) directing a pump light beam in a first direction x through a vapor cell comprising gaseous atoms;

    II) applying a (longitudinal) magnetic field component to the vapor cell parallel or antiparallel to the first direction x with different magnitudes $B_{x,i}^{app}$ ($i = 1, ..,$ N, N being an integer indicating the number of different magnitudes);

III) detecting light of the pump light beam directed through the vapor cell for the different magnitudes $B_{x,i}^{app}$ of the magnetic field component applied parallel or antiparallel to the first direction x;

IV) determining the maximum optical transmission $T_{max}$ and the minimum optical transmission $T_{min}$ based on the detected light;

V) determining the difference between the maximum optical transmission and the minimum optical transmission $D = T_{max} - T_{min}$;

VI) determining the magnitude of the x-component of an ambient magnetic field $B_x^{ext}$ by determining the particular magnitude $B_x^{app}$ of the x-component of the applied magnetic field for which the minimum optical transmission $T_{min}$ is determined;

VII) repeating at least I) to V) while applying (transversal) magnetic field components to the vapor cell in second and third directions y and z perpendicular to the first direction x with different magnitudes $B_{y,i}^{app}$ and $B_{z,i}^{app}$; and

VIII) determining the magnitudes $B_y^{ext}$ and $B_z^{ext}$ of the ambient magnetic field components in the second and third directions y and z by determining the particular magnitudes of the y- and z-components of the applied magnetic field $B_y^{app}$ and $B_z^{app}$ for which the difference between the maximum optical transmission and the minimum optical transmission D is minimized.

According to an implementation, the magnitudes $B_{y,i}^{app}$ and $B_{z,i}^{app}$ of the magnetic field components in the second and third directions y and z are varied in step VII) performed by the processing and controlling means based on at least one of a raster scan, spiral search starting from a starting magnitude and moving outward in a spiral pattern, Lissajous search with magnitudes oscillating sinusoidally at different frequencies and Rose curve search by oscillating the transversal applied magnetic field magnitudes $\sqrt{\left(B_y^{\mathrm{app}}\right)^2 + \left(B_z^{\mathrm{app}}\right)^2}$ sinusoidally while linearly scanning the angle $\arctan\left(B_z^{\mathrm{app}}/B_y^{\mathrm{app}}\right)$.

According to an implementation, in step VIII) performed by the processing and controlling means the magnitudes $B_y^{\mathrm{ext}}$ and $B_z^{\mathrm{ext}}$ of the ambient transversal magnetic field components are determined by weighted averages of the magnitudes $B_{y,i}^{\mathrm{app}}$ and $B_{z,i}^{\mathrm{app}}$ of the applied transversal magnetic field components wherein the weights are functions of the determined optical transmissions. The magnitudes $B_y^{\mathrm{ext}}$ and $B_z^{\mathrm{ext}}$ of the ambient transversal magnetic field components may be determined by

$$B_y^{\mathrm{ext}} = \sum_{i=1}^{n} B_{y,i}^{\mathrm{app}} \quad w_i,$$

$$B_z^{\mathrm{ext}} = \sum_{i=1}^{n} B_{z.i}^{\mathrm{app}} \quad w_i,$$

with

$$w_i = \begin{cases} \left(\dfrac{T_i}{T_{max}}\right)^x & T_i > v \ T_{max} \\ 0 & \text{otherwise} \end{cases}$$

wherein $T_{max}$ is the maximum value of the determined transmission $T_i$, $x$ is the power-law exponent, $v$ is the threshold fraction and i = 1, .., n, n being an integer indicating the number of different magnitudes.

According to an implementation, the light source is a laser device configured for generating the pump (laser) light beam.

According to an implementation, the apparatus further comprises a magnetic shielding configured for substantially shielding an external magnetic field from the vapor cell. The components of the ambient magnetic field determined as described above, in this case, are the components of the remaining external magnetic field remaining after magnetic shielding.

The apparatus and any implementation thereof provide respective similar advantages as discussed above with respect to the provided method and implementations thereof. The provided method and any implementation thereof may be performed by means of the provided apparatus and any implementation thereof.

Furthermore, it is provided a zero-field resonance optically pumped magnetometer comprising the apparatus according to any of the above-described examples. Very accurate measurement of 3D magnetic fields can be provided by such a zero-field resonance optically pumped magnetometer.

Additional features and advantages of the present invention will be described with reference to the drawings. In the description, reference is made to the accompanying figures that are meant to illustrate preferred embodiments of the invention. It is understood that such embodiments do not represent the full scope of the invention.

## Brief description of the drawings

Figure 1 schematically illustrates components and operation of an apparatus for detecting a zero-field resonance of an exemplary embodiment of the invention wherein a laser beam optically pumps spins of an atomic species and the optical transmission of the laser beam is detected while sweeping an applied magnetic field component orientated parallel or anti-parallel to the direction of propagation of the laser beam.

Figures 2a and b represent a flow chart illustrating a method of detecting a zero-field resonance in accordance with an exemplary embodiment of the invention.

Figure 3 illustrates a comparison of an approach of the prior art based on minimizing the width and maximize the height of the zero-field resonance (a) and an approach based on minimizing the transmission depth according to an embodiment (b).

Figure 4 illustrates the effect of small variations of applied transversal magnetic field components on depths of optical transmission dips.

## Description of specific embodiments of the invention

The present invention provides a method of detecting a zero-field resonance (ZFR) and an apparatus for detecting a zero-field resonance based on scanning/sweeping an applied magnetic field component orientated parallel or anti-parallel to the direction of propagation of a laser beam pumping spins of an atomic species contained in a vapor cell.

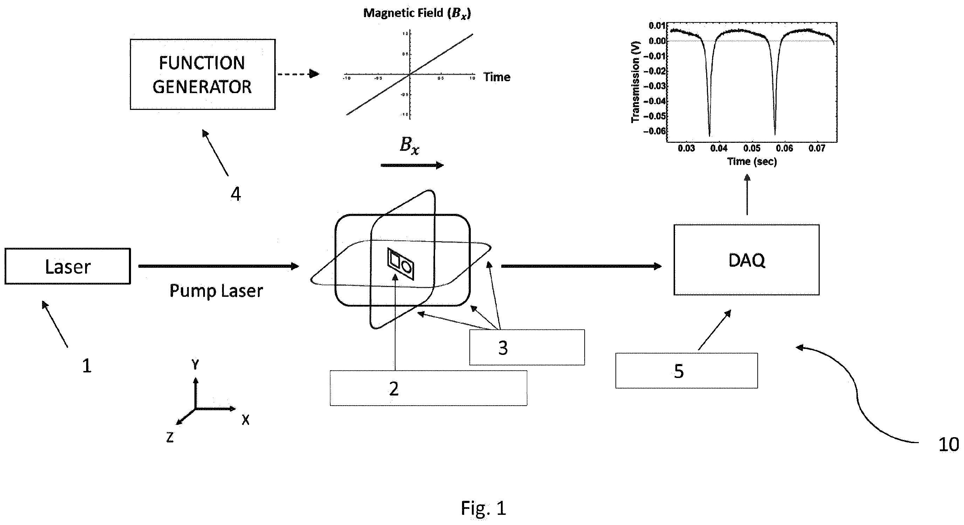

Figure 1 schematically illustrates components of an apparatus 10 for detecting a zero-field resonance according to an embodiment. The apparatus 10 comprises a laser device 1. The laser device 1 may be a Vertical Cavity Surface Emitting Laser (VCSEL) with an emission wavelength of about 795 nm (D1 line of $^{87}$Rb), for example.

The laser device 1 is used to generate a pump light (laser) beam in order to optically pump (the spins of) atoms contained in a vapor cell 2. For example, the pump light beam may generated by the laser device 1 at a power level of the order of $\mu$W. In general, the wavelength of the light emitted by the laser device 1 has to be adapted to the wavelength of a D1 or D2 line atomic transition of the atoms. The atomic medium contained in the vapor cell 2 is largely transparent to laser light at zero or near-zero magnetic field and is largely opaque to laser light at other values of the magnetic field (Zero-field resonance (ZFR) effect). For example, the atomic medium may comprise or consists of an alkali metal vapor.

In the example shown in Figure 1 the pump light beam enters the vapor cell 2 in the x-direction (beam propagation direction). The same pump light beam is used for monitoring the optical transmission through the vapor cell 2. For example, the pump light beam originating from the laser device 1 is collimated and guided to the vapor cell over a free space path of about 10 cm. A mirror, half-wave plate, a polarizing beam splitter cube and a quarter wave plate (not shown in Figure 1) may be used to circularly polarize the pump light beam and direct it through the vapor cell 2.

According to an embodiment, a buffer gas, for example, helium, neon or nitrogen, is provided in the vapor cell 2 in order to slow down the rate at which the atoms (for example, atoms of an alkali metal vapor) collide with the inner walls of the vapor cell 2 such that randomization of the spins of the alkali metal atoms caused by such collisions can be suppressed. The buffer gas pressure in the vapor cell 2 may be, for example, in a range of about 1 to 5 bars at room temperature but the method is equally applicable for any buffer gas density.

To increase the number density of the atoms of the active atomic medium the vapor cell 2 may be heated, for example, up to a temperature over 100°C, for example, 150 °C to 200 °C. For example, the vapor cell 2 may be located in an oven or a separate heating device may be provided for heating the active atomic medium. For example, a density of the active atomic medium in the range of $10^7$ cm$^{-3}$ to $10^{15}$ cm$^{-3}$ may be considered appropriate. According to a particular embodiment, the vapor cell 2 is filled with isotopically enriched Rubidium-87 and a nitrogen buffer gas obtained through the process of Rubidium-azide (RbN$_3$) decomposition caused by irradiation of UV light. Besides rubidium other suitable active atomic media may consist of or comprise cesium, potassium or sodium.

The vapor cell 2 may be sealed through anodic bonding of borosilicate glass to silicon. Special coatings on the inner walls of the vapor cell 2, such as octadecyltrichlorosilane (OTS) or paraffin, may be provided to reduce spin randomization from wall collisions of the active atomic medium. All components of the housing of the vapor cell 2 should be chosen to be either non-magnetic or to have a very low residual magnetization.

The apparatus 10, furthermore, comprises a set of (electrically activated biasing) biplanar magnetic field generating coils 3 with optimized current traces for generating a homogeneous magnetic field (for example, with a strength in the range of a few 10 nT to 100 nT or a few 100 nT or of the order of $\mu$T) at the position of the vapor cell 2 (to cancel out/compensate for some (residual) ambient magnetic field) and minimum stray fields around the module. The coils 3 may be configured to provide a magnetic field with a strength in order of an ambient magnetic field (or, after shielding, a residual ambient magnetic field). The vapor cell 2 is positioned in between the biplanar coils 3 and the whole module may be placed inside a four-layer magnetic shield housing giving raise to some residual ambient magnetic field being present at the location of the active atomic medium. The shield housing may be comprised of 3 layers of mu-metal (non-oriented nickel-iron soft ferromagnetic alloy) and one layer of ferrite, but any material that can suitably shield the ambient magnetic noise may equally be used acceptable. Further, only a partially shielding housing or no shield housing at all may be present according to different embodiments and applications.

The apparatus 10, furthermore, comprises a function generator (processing means) 4 configured for linearly sweeping/scanning (for example, from some predetermined negative value to some predetermined positive value, see sketch above the vapor cell 2 in Figure 1) the applied magnetic field component $B_x^{\mathrm{app}}$ that is orientated parallel or antiparallel to the beam propagation direction x. The function generator 4 may control magnitudes and directions of electric currents applied to one of the coils 3 in order to perform the sweeping/scanning of the magnetic field component $B_x$. Further, the function generator 4 may be configured to alter applied y- and z-components of the magnetic field $B_y^{\mathrm{app}}$ and $B_z^{\mathrm{app}}$ at the location of the vapor cell 2 (again, by controlling the application of currents to the corresponding ones of the coils 3).

The apparatus 10, furthermore, comprises a detector device 5 configured for detecting transmission of the pump light beam through the (active atomic medium of) the vapor cell 2. The detector device 5 may be or comprises an Si photodetector and may be supplemented by some (for example, photodiode) signal amplifier. Particularly, the detector device 5 is configured for detecting a ZFR signal defined by the optical transmission $T(B_x, B_y, B_z)$ as a function of the magnetic field at the vapor cell 2 $B = B^{\mathrm{ext}} - B^{\mathrm{app}}$ given by the difference of the ambient magnetic vector field and the applied magnetic vector field, and, more particularly, for detecting optical transmission dips (see sketch above the detector device

Figure 1 and description below). It is noted that the transmission T as a function of the applied magnetic field $(B_x^{app}, B_y^{app}, B_z^{app})$ has the same shape as the transmission T as a function of the magnetic field at the vapor cell 2 $T(B_x, B_y, B_z)$ with a center at $B^{ext}$.

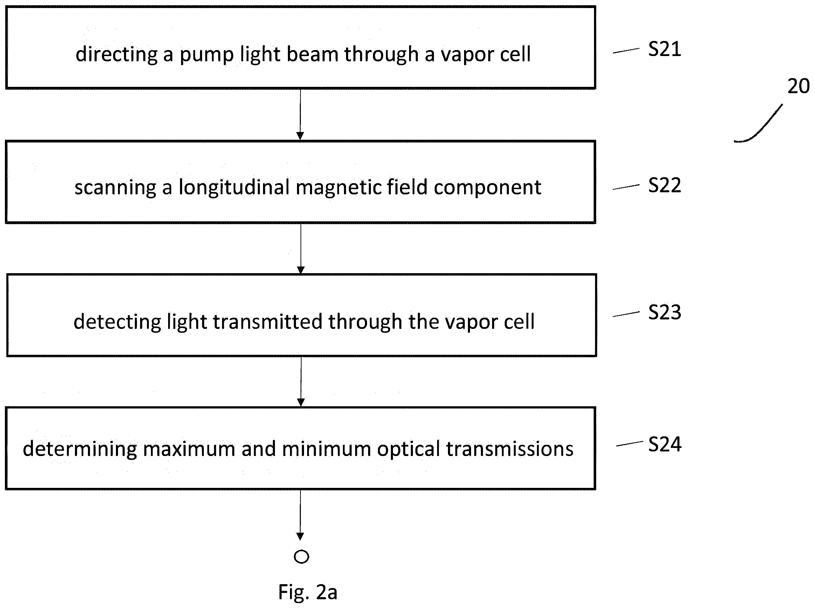

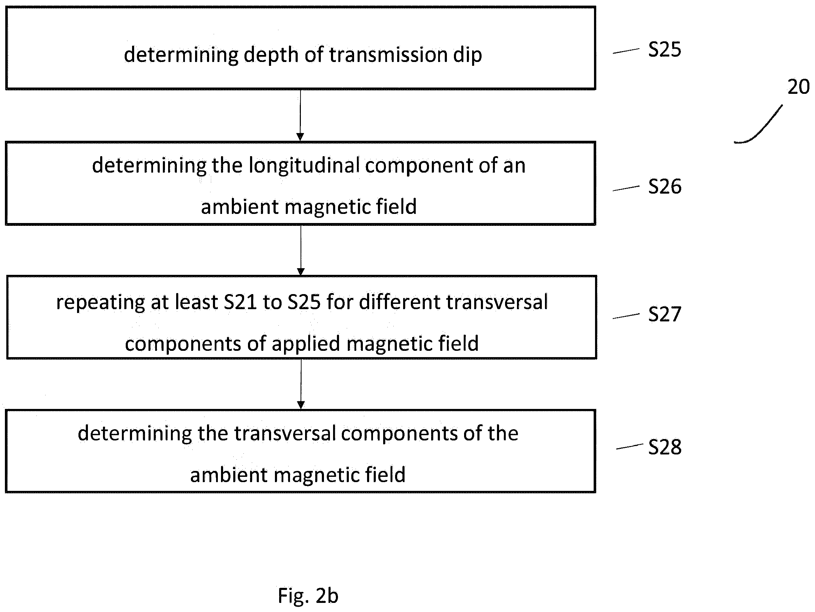

Figures 2a and 2b represent a flow chart illustrating a method 20 of detecting a zero-field resonance (ZFR) in accordance with an exemplary embodiment of the invention. For example, the method 20 can be carried out by means of the apparatus 10 illustrated in Figure 1. Whereas the steps of the method 20 are shown in a particular order for illustration purposes individual steps may be carried out concurrently or even in a different order where considered appropriate.

In step S21 of the method 20 illustrated in Figure 2a a pump light beam is directed in a first direction x through a vapor cell comprising gaseous atoms. The pump light beam may be generated by the laser device 1 shown in Figure 1. The vapor cell may be the vapor cell 2 shown in Figure 1. The gaseous atoms may comprise one of alkali atoms, rubidium atoms, cesium atoms, potassium atoms, sodium atoms and helium atoms. The vapor cell may further contain a buffer gas.

A magnetic field component is applied S22 to the vapor cell parallel or antiparallel to the first direction x with different magnitudes $B_{x,i}^{app}$, i = 1, .., N, N being an integer indicating the number of different magnitudes. This step of sweeping/scanning the longitudinal component of the applied magnetic field (i.e., the component parallel/antiparallel to the direction of propagation of the pump light beam) with N different magnitudes is essential for the present invention.

The method 20 illustrated in Figure 2a, further, comprises detecting S23 light of the pump light beam directed through the vapor cell for the different magnitudes $B_{x,i}^{app}$ of the magnetic field component applied parallel or antiparallel to the first direction x and determining S24 the maximum optical transmission $T_{max}$ and the minimum optical transmission $T_{min}$ based on the detected light. The depth D of the dip of the transmission T is determined by determining S25 the difference between the maximum optical transmission and the minimum optical transmission determined in step S24: D = $T_{max}$ - $T_{min}$. It is noted that $T_{max}$ and $T_{min}$ and, thus, the depth D of the dip are also functions of the transversal components of the applied magnetic field $B_y^{app}$ and $B_z^{app}$. The magnitude of the x-component of an ambient magnetic field $B_x^{ext}$ is determined in step S26 by determining the particular magnitude $B_x^{appl}$ of the x-component of the applied magnetic field for which the minimum optical transmission $T_{min}$ is obtained. This magnitude $B_x^{ext}$ of the x-component of the ambient magnetic field may be considered to be equal to the particular magnitude $B_x^{app}$ of the N magnitudes of the magnetic field component applied parallel or antiparallel to the first direction x in step S22 for which the minimum optical transmission $T_{min}$ is obtained.

As already said $T_{max}$ and $T_{min}$ and, thus, the depth D of the dip are also functions of the transversal components of the applied magnetic field $B_y^{app}$ and $B_z^{app}$. Therefore, at least steps S21 to S25 are repeated S27 while applying (transversal) magnetic field components $B_y^{app}$ and $B_z^{app}$ to the vapor cell in second and third directions y and z perpendicular to the first direction x with different magnitudes $B_{y,i}^{app}$ and $B_{z,i}^{app}$ and the magnitudes $B_y^{ext}$ and $B_z^{ext}$ of the ambient magnetic field components in the second and third directions y and z are determined S28 by determining the y- and z-components of the applied magnetic field for which the difference between the maximum optical transmission and the minimum optical transmission D is minimized. These magnitudes $B_y^{ext}$ and $B_z^{ext}$ of the ambient magnetic field components in the second and third directions y and z may be considered to be equal to or may be determined based on the particular ones $B_y^{app}$ and $B_z^{app}$ of the N magnitudes of the applied transversal magnetic field components for which the difference between the maximum optical transmission and the minimum optical transmission D is minimized. It is noted that when carrying out step S22 the first time before the repetition cycle S27 the y- and z-components of the applied magnetic field may be chosen to be zero. Further, when repeating step S22 during the repetition cycle S27 the number of different magnitudes N of the applied longitudinal magnetic field component may be varied with respect to the first performance of step S22 before the repetition cycle S27. According to a particular example, N=1 during the repetition cycle S27 and the only applied longitudinal magnetic field component has the particular magnitude $B_x^{app}$ determined in step S26.

The magnitudes $B_{y,i}^{\mathrm{app}}$ and $B_{z,i}^{\mathrm{app}}$ of the magnetic field components in the second and third directions y and z may be varied in step S27 based on at least one of a

  a. raster scan
  b. spiral search starting from a starting magnitude and moving outward in a spiral pattern
  c. Lissajous search with magnitudes oscillating sinusoidally at different frequencies; and
  d. Rose curve search by oscillating the transversal applied magnetic field magnitudes

$$\sqrt{\left(B_y^{\mathrm{app}}\right)^2 + \left(B_z^{\mathrm{app}}\right)^2}$$

sinusoidally while linearly scanning the angle $\arctan\left(B_z^{\mathrm{app}}/B_y^{\mathrm{app}}\right)$

The magnitudes $B_y^{\mathrm{ext}}$ and $B_z^{\mathrm{ext}}$ of the ambient transversal magnetic field components may be determined is step S28 by weighted averages of the magnitudes $B_{y,i}^{\mathrm{app}}$ and $B_{z,i}^{\mathrm{app}}$ of the applied transversal magnetic field components wherein the weights are functions of the determined optical transmissions. For example, the magnitudes $B_y^{\mathrm{ext}}$ and $B_z^{\mathrm{ext}}$ of the ambient transversal magnetic field components are determined by

$$B_y^{\mathrm{ext}} = \sum_{i=1}^n B_{y,i}^{\mathrm{app}} \quad w_i,$$

$$B_z^{\mathrm{ext}} = \sum_{i=1}^n B_{z,i}^{\mathrm{app}} \quad w_i,$$

with

$$w_i = \begin{cases} \left(\dfrac{T_i}{T_{max}}\right)^x & T_i > v \ T_{max} \\ 0 & \text{otherwise} \end{cases}$$

wherein $T_{max}$ is the maximum value of the determined transmission $T_i$, $x$ is a power-law exponent, $v$ is the threshold fraction and i = 1, .., n, n being an integer indicating the number of different magnitudes.
It is noted that step S26 of determining the magnitude of the x-component of an ambient magnetic field $B_x^{\mathrm{ext}}$ by determining the particular magnitude $B_x^{\mathrm{app}}$ of the x-component of the applied magnetic field for which the minimum optical transmission $T_{min}$ is de-

termined may also be repeated through the cycle of step S27 and, for example, the x-component of an ambient magnetic field $B_x^{\mathrm{ext}}$ may be determined as an average of the particular magnitudes $B_x^{\mathrm{app}}$ of the x-component of the applied magnetic field for which the minimum optical transmission $T_{min}$ is determined for different magnitudes of the y- and z-components of the applied magnetic field $B_y^{\mathrm{app}}$ and $B_z^{\mathrm{app}}$.

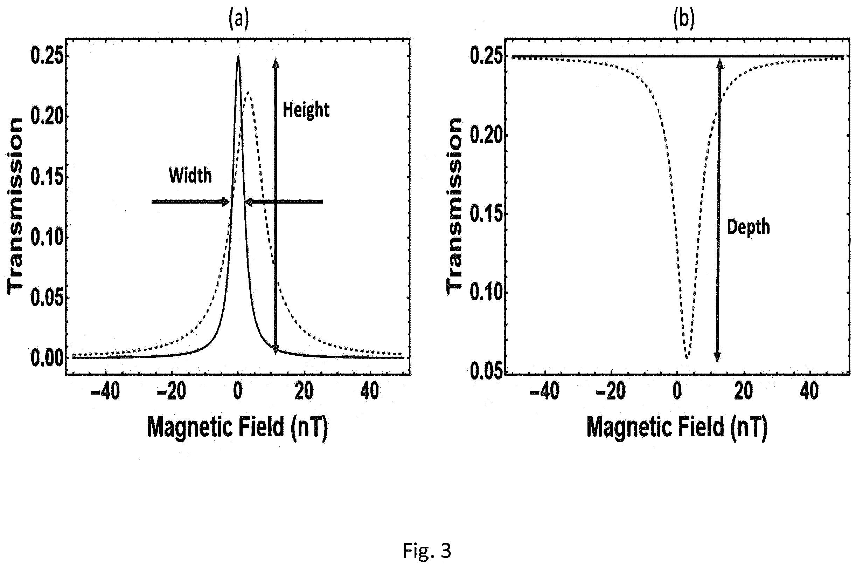

As described above, according to the invention an applied longitudinal magnetic field component is scanned and the depth D of a transmission dip (the difference between the maximum optical transmission and the minimum optical transmission) is determined S25 and the depth D is minimized S28 to adjust $B_y^{\mathrm{app}}$ and $B_z^{\mathrm{app}}$ to the ambient field to cancel it out at the location of the vapor cell. This procedure is different from the art exemplified by US 2016/0223627 A1 as it is illustrated in Figure 3. US 2016/0223627 A1 proposes application of a bias magnetic field in the direction of the laser pump beam to simultaneously increase both the height and width of the ZFR and, after detection of the ZFR, a magnetic field is scanned/swept in directions perpendicular to that of the pump light beam (transversal directions of the magnetic field) and these transversal magnetic field components are adjusted to minimize the width and maximize the height of the zero-field resonance (see left column (a) of Figure 3 showing optical transmission versus a transversal magnetic field component). Contrary, according to the invention optical transmission as a function of a scanned longitudinal magnetic field component is swept through zero (see right column (b) of Figure 3 showing optical transmission versus the longitudinal magnetic field component).

As compared to the art, the inventive approach provides the advantage that optimization has to be with respect to a substantially non-zero value of the depth D of a transmission dip which is possible with a relatively low calculation precision needed as compared to an optimization with respect to relatively large finite values of the height and width of the ZFR wherein the detection of small changes of the relatively large measuring quantities have to be considered. Particularly, significantly shorter averaging times for obtaining reasonably useful measurement results are needed according to the inventive method and, thus, the scan speed and overall processing speed can be increased. Further, accuracy of the determined ambient magnetic field is increased by the inventive method.

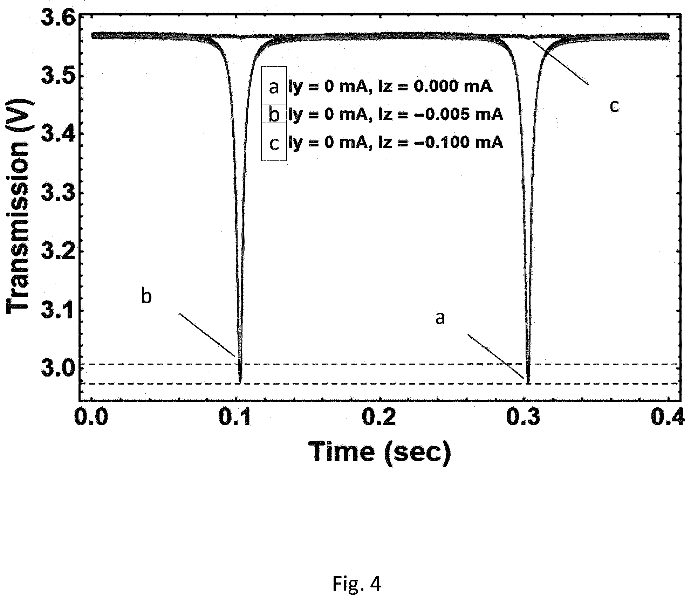

As it is illustrated in Figure 4 even a small variation of the current applied to a coil providing applied transversal magnetic field components has a relatively large effect on the depth of the dip (D) of the optical transmission. In the example shown in Figure 4 the optical transmission measured in V is plotted against the longitudinal scanning time in s (and, thus, variation in the applied longitudinal magnetic field component) for coil currents for the application of the transversal magnetic field components of Iy= 0 mA and Iz = 0.000 mA (curve a), Iy= 0 mA and Iz = -0.005 mA (curve b), and Iy= 0 mA and Iz = -0.100 mA (curve c), respectively. The minimum of D is obtained for Iy= 0 mA and Iz = 0.000 mA (curve a). Even a small variation of magnitude of the applied current of 0.005 mA has a significant effect on D close to the zero field (see curve b). Thus, adjustment to the ambient field can be achieved quickly and accurately.

The inventive method and apparatus, for example, the embodiments illustrated in Figures 1 and 2a and 2b can be advantageously used in a great variety of applications including optically pumped magnetometers (OPMs), for example, in the context of magnetoencephalography (a medical imaging technique that provides functional information about the brain and is used for presurgical characterization of epilepsy and for diagnosis of mild traumatic brain injury) or in the context of the characterization of batteries, e.g., for transportation, wherein ZFR-OPMs are used to non-invasively detect and localize leakage currents and diagnose battery health. Other applications include magnetic navigation using the Earth's field as a reference and magnetic communication for underwater and underground vehicles and installations, geotechnical and minerals exploration techniques, and clinical and pre-clinical magnetic resonance investigations wherein ZFR-OPMs are used together with 'hyperpolarized' nuclear spins in molecules used, for example, as tracer compounds for metabolism studies in patients.

All previously discussed embodiments are not intended as limitations but serve as examples illustrating features and advantages of the invention. It is to be understood that some or all of the above-described features can also be combined in different ways.

## Acknowledgement

This work has been partially funded by the European Union's Horizon Europe research and innovation programme under grant agreement No. 101099379, by the Secretariat of Digital Policies of the Government of Catalonia - G.A. GOV/51/2022, and has received the support of a fellowship from "la Caixa" Foundation (ID 100010434), under the fellowship code LCF/BQ/DI24/12070013.

## Claims

1. Method (20) of detecting a zero-field resonance, comprising the steps of

    i) directing (S21) a pump light beam in a first direction x through a vapor cell comprising gaseous atoms;
    ii) applying (S22) a magnetic field component to the vapor cell parallel or anti-parallel to the first direction x with different magnitudes $B_{x,i}^{\mathrm{app}}$;
    iii) detecting (S23) light of the pump light beam directed through the vapor cell for the different magnitudes $B_{x,i}^{\mathrm{app}}$ of the magnetic field component applied parallel or antiparallel to the first direction x;
    iv) determining (S24) the maximum optical transmission $T_{\mathrm{max}}$ and the minimum optical transmission $T_{\mathrm{min}}$ based on the detected light;
    v) determining (S25) the difference between the maximum optical transmission and the minimum optical transmission $D = T_{\mathrm{max}} - T_{\mathrm{min}}$;
    vi) determining (S26) the magnitude of the x-component of an ambient magnetic field $B_x^{\mathrm{ext}}$ by determining the particular magnitude $B_x^{\mathrm{app}}$ of the x-component of the applied magnetic field for which the minimum optical transmission $T_{\mathrm{min}}$ is determined;
    vii) repeating (S27) at least steps i) to v) while applying magnetic field components to the vapor cell in second and third directions y and z perpendicular to the first direction x with different magnitudes $B_{y,i}^{\mathrm{app}}$ and $B_{z,i}^{\mathrm{app}}$; and
    viii) determining (S28) the magnitudes $B_y^{\mathrm{ext}}$ and $B_z^{\mathrm{ext}}$ of the ambient magnetic field components in the second and third directions y and z by determining the particular magnitudes of the y- and z-components of the applied magnetic field $B_y^{\mathrm{app}}$ and $B_z^{\mathrm{app}}$ for which the difference between the maximum optical transmission and the minimum optical transmission D is minimized.

2. The method (20) according to claim 1, wherein the magnitudes $B_{y,i}^{\mathrm{app}}$ and $B_{z,i}^{\mathrm{app}}$ of the magnetic field components in the second and third directions y and z are varied in step vii) based on at least one of a

    a. raster scan
    b. spiral search starting from a starting magnitude and moving outward in a spiral pattern
    c. Lissajous search with magnitudes oscillating sinusoidally at different frequencies; and

d. Rose curve search.

3. The method (20) according to claim 1 or 2, wherein in step viii) the magnitudes $B_y^{\text{ext}}$ and $B_z^{\text{ext}}$ of the ambient transversal magnetic field components are determined (S28) by weighted averages of the magnitudes $B_{y,i}^{\text{app}}$ and $B_{z,i}^{\text{app}}$ of the applied transversal magnetic field components wherein the weights are functions of the determined optical transmissions.

4. The method (20) according to claim 3, wherein the magnitudes $B_y^{\text{ext}}$ and $B_z^{\text{ext}}$ of the ambient transversal magnetic field components are determined by

$$B_y^{\text{ext}} = \sum_{i=1}^{n} B_{y,i}^{\text{app}} \; w_i,$$

$$B_z^{\text{ext}} = \sum_{i=1}^{n} B_{z,i}^{\text{app}} \; w_i,$$

with

$$w_i = \begin{cases} \left(\dfrac{T_i}{T_{max}}\right)^x & T_i > v \; T_{max} \\ 0 & \text{otherwise} \end{cases}$$

wherein $T_{max}$ is the maximum value of the determined transmission $T_i$, $x$ is a power-law exponent, $v$ is the threshold fraction and i = 1, .., n, n being an integer indicating the number of different magnitudes.

5. The method (20) according to one of the preceding claims, further comprising generating the pump light beam by a laser.

6. The method (20) according to one of the preceding claims, wherein the gaseous atoms are or comprise one of alkali atoms, rubidium atoms, cesium atoms, potassium atoms, sodium atoms and helium atoms.

7. An apparatus (10) for detecting a zero-field resonance, comprising

a vapor cell (2) comprising gaseous atoms;
a light source (1) configured to direct a pump light beam in a first direction x through the vapor cell (2);
magnetic coils (3) arranged and configured to apply a magnetic field to the vapor cell (2);

a detector device (5) configured to detect light of the pump light beam directed through the vapor cell; and
a processing and controlling means configured to perform

I) directing a pump light beam in a first direction x through a vapor cell comprising gaseous atoms;
II) applying a magnetic field component to the vapor cell parallel or antiparallel to the first direction x with different magnitudes $B_{x,i}^{\text{app}}$;
III) detecting light of the pump light beam directed through the vapor cell for the different magnitudes $B_{x,i}^{\text{app}}$ of the magnetic field component applied parallel or antiparallel to the first direction x;
IV) determining the maximum optical transmission $T_{\text{max}}$ and the minimum optical transmission $T_{\text{min}}$ based on the detected light;
V) determining the difference between the maximum optical transmission and the minimum optical transmission D = $T_{\text{max}}$-$T_{\text{min}}$;
VI) determining the magnitude of the x-component of an ambient magnetic field $B_x^{\text{ext}}$ by determining the particular magnitude $B_x^{\text{app}}$ of the x-component of the applied magnetic field for which the minimum optical transmission $T_{\text{min}}$ is determined;
VII) repeating at least I) to V) while applying magnetic field components to the vapor cell in second and third directions y and z perpendicular to the first direction x with different magnitudes $B_{y,i}^{\text{app}}$ and $B_{z,i}^{\text{app}}$; and
VIII) determining the magnitudes $B_y^{\text{ext}}$ and $B_z^{\text{ext}}$ of the ambient magnetic field components in the second and third directions y and z by determining the particular magnitudes of the y- and z-components of the applied magnetic field $B_y^{\text{app}}$ and $B_z^{\text{app}}$ for which the difference between the maximum optical transmission and the minimum optical transmission D is minimized.

8. The apparatus (10) according to claim 7, wherein the magnitudes $B_{y,i}^{\mathrm{app}}$ and $B_{z,i}^{\mathrm{app}}$ of the magnetic field components in the second and third directions y and z are varied in step VII) performed by the processing and controlling means based on at least one of a

    a. raster scan
    b. spiral search starting from a starting magnitude and moving outward in a spiral pattern
    c. Lissajous search with magnitudes oscillating sinusoidally at different frequencies; and
    d. Rose curve search.

9. The apparatus (10) according to claim 7 or 8, wherein in step VIII) performed by the processing and controlling means the magnitudes $B_y^{\mathrm{ext}}$ and $B_z^{\mathrm{ext}}$ of the ambient transversal magnetic field components are determined by weighted averages of the magnitudes $B_{y,i}^{\mathrm{app}}$ and $B_{z,i}^{\mathrm{app}}$ of the applied transversal magnetic field components wherein the weights are functions of the determined optical transmissions.

10. The apparatus (10) according to claim 9, wherein the magnitudes $B_y^{\mathrm{ext}}$ and $B_z^{\mathrm{ext}}$ of the ambient transversal magnetic field components are determined by

$$B_y^{\mathrm{ext}} = \sum_{i=1}^{n} B_{y,i}^{\mathrm{app}} \; w_i,$$

$$B_z^{\mathrm{ext}} = \sum_{i=1}^{n} B_{z,i}^{\mathrm{app}} \; w_i,$$

with

$$w_i = \begin{cases} \left(\frac{T_i}{T_{max}}\right)^x & T_i > v \; T_{max} \\ 0 & \text{otherwise} \end{cases}$$

wherein $T_{max}$ is the maximum value of the determined transmission $T_i$, x is the power-law exponent, v is the threshold fraction and i = 1, .., n, n being an integer indicating the number of different magnitudes.

11. The apparatus (10) according any of the claims 7 to 10, wherein the light source (1) is a laser device (1) configured for generating the pump light beam.

12. The apparatus (10) according to any of the claims 7 to 11, wherein the gaseous atoms are or comprise one of alkali atoms, rubidium atoms, cesium atoms, potassium atoms, sodium atoms and helium atoms.

13. The apparatus (10) according to any of the claims 7 to 12, further comprising a magnetic shielding configured for substantially shielding an external magnetic field from the vapor cell.

14. A zero-field resonance optically pumped magnetometer comprising the apparatus (10) according to any of the claims 7 to 13.

EP 4 682 569 A1

FUNCTION GENERATOR

Magnetic Field ($B_x$)

Time

$B_x$

Transmission (V)

Time (sec)

Laser

Pump Laser

DAQ

1

4

2

3

5

10

Y

X

Z

Fig. 1

20

S21 directing a pump light beam through a vapor cell

S22 scanning a longitudinal magnetic field component

S23 detecting light transmitted through the vapor cell

S24 determining maximum and minimum optical transmissions

Fig. 2a

```
┌─────────────────────────────────────────────┐
│                                             │
│    determining depth of transmission dip    │ ── S25
│                                             │
└─────────────────────────────────────────────┘
                      │                                          20
                      ▼
┌─────────────────────────────────────────────┐
│   determining the longitudinal component of an │ ── S26
│          ambient magnetic field             │
└─────────────────────────────────────────────┘
                      │
                      ▼
┌─────────────────────────────────────────────┐
│  repeating at least S21 to S25 for different transversal │ ── S27
│       components of applied magnetic field   │
└─────────────────────────────────────────────┘
                      │
                      ▼
┌─────────────────────────────────────────────┐
│    determining the transversal components of the │ ── S28
│           ambient magnetic field             │
└─────────────────────────────────────────────┘
```

Fig. 2b

Fig. 3

Fig. 4

**EUROPEAN SEARCH REPORT**

Europäisches
Patentamt
European
Patent Office
Office européen
des brevets

Application Number

EP 24 38 2785

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A,D | US 2016/223627 A1 (SHAH VISHAL [US] ET AL) 4 August 2016 (2016-08-04) * the whole document * | 1-14 | INV. G01R33/26 G01N24/00 |
| A | CN 109 738 837 A (UNIV BEIHANG) 10 May 2019 (2019-05-10) * the whole document * | 1-14 | |
| A | GUO YANG ET AL: "Active Magnetic Compensation Based on Parametric Resonance Magnetometer", JOURNAL OF SHANGHAI JIAOTONG UNIVERSITY (ENGLISH EDITION), [Online] vol. 29, no. 2, 12 November 2022 (2022-11-12), pages 280-289, XP093236272, Heidelberg ISSN: 1007-1172, DOI: 10.1007/s12204-022-2524-4 Internet Retrieved from the Internet: URL:https://link.springer.com/article/10.1 007/s12204-022-2524-4/fulltext.html> [retrieved on 2024-12-13] * the whole document * | 1-14 | TECHNICAL FIELDS SEARCHED (IPC) G01N G01R A61B |
| A | ZHANG SHAOWEN ET AL: "Triaxial precise magnetic field compensation of a zero-field optically pumped magnetometer based on a single-beam configuration", OPTICS EXPRESS, vol. 30, no. 14, 22 June 2022 (2022-06-22), page 24579, XP093236273, US ISSN: 1094-4087, DOI: 10.1364/OE.464361 * the whole document * | 1-14 | |

The present search report has been drawn up for all claims

| Place of search | Date of completion of the search | Examiner |
|---|---|---|
| Munich | 2 January 2025 | Skalla, Jörg |

CATEGORY OF CITED DOCUMENTS

X : particularly relevant if taken alone
Y : particularly relevant if combined with another
    document of the same category
A : technological background
O : non-written disclosure
P : intermediate document

T : theory or principle underlying the invention
E : earlier patent document, but published on, or
    after the filing date
D : document cited in the application
L : document cited for other reasons
...................................................................................
& : member of the same patent family, corresponding
    document

EPO FORM 1503 03.82 (P04C01)

## ANNEX TO THE EUROPEAN SEARCH REPORT
## ON EUROPEAN PATENT APPLICATION NO.

EP 24 38 2785

This annex lists the patent family members relating to the patent documents cited in the above-mentioned European search report.
The members are as contained in the European Patent Office EDP file on
The European Patent Office is in no way liable for these particulars which are merely given for the purpose of information.

02-01-2025

| Patent document cited in search report | | Publication date | Patent family member(s) | | Publication date |
|---|---|---|---|---|---|
| US 2016223627 | A1 | 04-08-2016 | US | 2015212168 A1 | 30-07-2015 |
| | | | US | 2016223627 A1 | 04-08-2016 |
| CN 109738837 | A | 10-05-2019 | NONE | | |

EPO FORM P0459

For more details about this annex : see Official Journal of the European Patent Office, No. 12/82

**EP 4 682 569 A1**